# EUROPÄISCHE PATENTANMELDUNG

(11) **EP 4 703 349 A1**
(43) Veröffentlichungstag der Anmeldung: **04.03.2026**
(21) Anmeldenummer: 24198043.2
(22) Anmeldetag: 03.09.2024
(51) Int. Cl.: C07C 69/608, C08K 5/00

(54) **TRIPENTYLESTER DER 1,2,4-CYCLOHEXANTRIPROPIONSÄURE UND DIESE ENTHALTENDE WEICHMACHERZUSAMMENSETZUNGEN**

(71) Anmelder: Evonik Oxeno GmbH & Co. KG, 45772 Marl (DE)
(72) Erfinder: GRASS, Michael, 45721 Haltern am See (DE); RUSEK, Monika, 45149 Essen (DE); VAN EICKELS, Martin, 45657 Recklinghausen (DE)
(74) Vertreter: Evonik Patent Association

(57) **Zusammenfassung**

Die vorliegende Erfindung betrifft Trialkylester der 1,2,4-Cyclohexantripropionsäure nach der Formel (1) 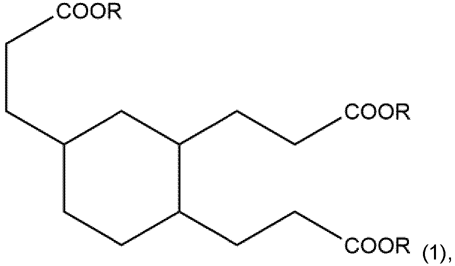 bei denen die drei Alkylgruppen R jeweils 5 Kohlenstoffatome aufweisen, von denen 55 bis 95 Mol% n-Pentylreste sind. Ein weiterer Gegenstand ist eine Weichmacherzusammensetzung, die die vorgenannten Trialkylester der 1,2,4-Cyclohexantripropionsäure nach der Formel (1) umfassen.

## Beschreibung

Gegenstand der Erfindung sind Trialkylester der 1,2,4-Cyclohexantripropionsäure nach der Formel (1) 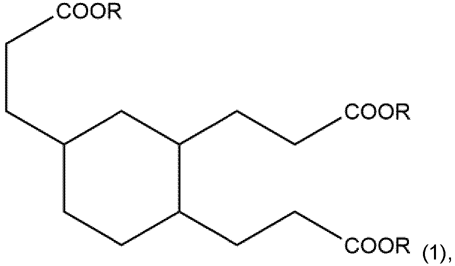 bei denen die drei Alkylgruppen R jeweils 5 Kohlenstoffatome aufweisen, von denen 55 bis 95 Mol% n-Pentylreste sind. Ein weiterer Gegenstand ist eine Weichmacherzusammensetzung, die die vorgenannten Trialkylester der 1,2,4-Cyclohexantripropionsäure nach der Formel (1) umfassen.

Die erfindungsgemäßen Verbindungen nach der Formel (1) sind Trialkylester der 1,2,4-Cyclohexantripropionsäure, wobei die drei Alkylgruppen jeweils 5 Kohlenstoffatome aufweisen. Trialkylester der 1,2,4-Cyclohexantripropionsäure sind grundsätzlich bekannt und beispielsweise in der EP 3 838 888 A1 beschrieben worden. Dort wurde auch erwähnt, dass diese Ester der 1,2,4-Cyclohexantripropionsäure als Weichmacher für Polymere eingesetzt werden können.

Auch wenn die Trialkylester der 1,2,4-Cyclohexantripropionsäure bekannt und ihr Einsatz als Weichmacher erwähnt worden ist, besteht ein kontinuierlicher Bedarf an einer Verbesserung der Eigenschaften beim Einsatz als Weichmacher. Je nach Anwendung können die Anforderungen an den einzusetzenden Weichmacher variieren.

Der vorliegenden Erfindung lag nun die Aufgabe zugrunde, eine Verbindung bereitzustellen, die gegenüber den bekannten Trialkylestern der 1,2,4-Cyclohexantripropionsäure verbesserte Eigenschaften aufweist. Zudem sollte eine Weichmacherzusammensetzung bereitgestellt werden, die Trialkylester der 1,2,4-Cyclohexantripropionsäure enthält, aber bessere anwendungstechnische Eigenschaften aufweist als die Zusammensetzungen mit bekannten Trialkylestern der 1,2,4-Cyclohexantripropionsäure.

Diese Aufgabe wird durch die speziellen Trialkylestern der 1,2,4-Cyclohexantripropionsäure nach Anspruch 1 gelöst. die Weichmacherzusammensetzung nach Anspruch 1 gelöst. Die Aufgabe wird weiterhin gelöst durch die Weichmacherzusammensetzung nach Anspruch 5. Bevorzugte Ausführungsformen sind in den abhängigen Ansprüchen angegeben.

Ein Gegenstand der vorliegenden Erfindung sind Trialkylester der 1,2,4-Cyclohexantripropionsäure nach der Formel (1) 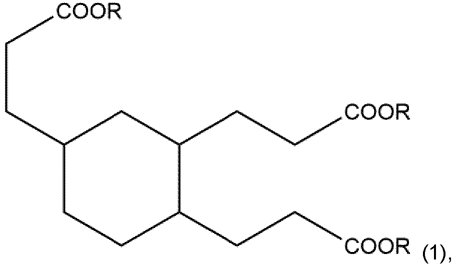 bei denen die drei Alkylgruppen R jeweils 5 Kohlenstoffatome aufweisen, von denen 55 bis 95 Mol% n-Pentylreste sind. In einer bevorzugten Ausführungsform sind 60 bis 95 Mol% der Alkylgruppen R n-Pentylreste. Die Prozentangabe Mol% bezieht sich auch hier jeweils auf 100 Mol% der Reste R, also alle Reste R der Verbindung nach Formel (1).

Die erfindungsgemäßen Verbindungen nach Formel (1) sind folglich Tripentylester der 1,2,4-Cyclohexantripropionsäure, der ein gewisser Anteil an einem oder mehreren Isomeren der Pentylgruppe vorhanden sind. Wichtigste Vertreter bei den Isomeren der Pentylgruppe sind die lineare n-Pentylgruppe und die einfach verzweigten 2-Methylbutylgruppe und 3-Methylbutylgruppe.

Es ist erfindungsgemäß bevorzugt, dass der Trialkylester der 1,2,4-Cyclohexantripropionsäure neben den n-Pentylresten ausschließlich 2-Methylbutyl- und/oder 3-Methylbutylreste umfasst. Somit sind vorzugsweise 5 bis 45 Mol%, vorzugsweise 5 bis 40 Mol% der Alkylgruppen R in den Trialkylestern der 1,2,4-Cyclohexantripropionsäure nach der Formel (1) 2-Methylbutylreste und/oder 3-Methylbutylreste.

Besonders bevorzugt enthalten die Alkylgruppen der Trialkylester der 1,2,4-Cyclohexantripropionsäure nach der Formel (1) neben den n-Pentylresten nur 2-Methylbutylreste. Somit sind vorzugsweise 5 bis 45 Mol%, vorzugsweise 5 bis 40 Mol% der Alkylgruppen R in den Trialkylestern der 1,2,4-Cyclohexantripropionsäure nach der Formel (1) 2-Methylbutylreste.

Ein weiterer Gegenstand der vorliegenden Erfindung ist eine Weichmacherzusammensetzung, die die vorher beschriebenen Trialkylester der 1,2,4-Cyclohexantripropionsäure enthält. Das entspricht einer Weichmacherzusammensetzung, enthaltend Trialkylester der 1,2,4-Cyclohexantripropionsäure nach der Formel (1) 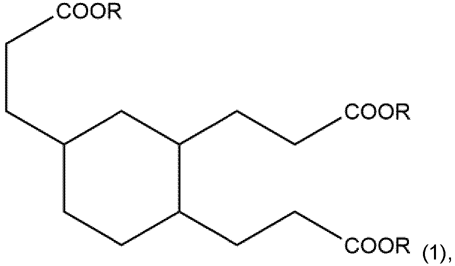 bei denen die drei Alkylgruppen R jeweils 5 Kohlenstoffatome aufweisen, von denen 55 bis 95 Mol%, vorzugsweise 60 bis 95 Mol% n-Pentylreste sind. Die Prozentangabe Mol% bezieht sich auch hier jeweils auf 100 Mol% der Reste R, also alle Reste R der Verbindung nach Formel (1).

Es ist erfindungsgemäß bevorzugt, dass der Trialkylester der 1,2,4-Cyclohexantripropionsäure in der Weichmacherzusammensetzung neben den n-Pentylresten ausschließlich 2-Methylbutyl- und/oder 3-Methylbutylreste umfasst. Somit sind vorzugsweise 5 bis 45 Mol%, vorzugsweise 5 bis 40 Mol% der Alkylgruppen R in den Trialkylestern der 1,2,4-Cyclohexantripropionsäure nach der Formel (1) 2-Methylbutylreste und/oder 3-Methylbutylreste.

Besonders bevorzugt enthalten die Alkylgruppen der Trialkylester der 1,2,4-Cyclohexantripropionsäure in der Weichmacherzusammensetzung nach der Formel (1) neben den n-Pentylresten nur 2-Methylbutylreste. Somit sind vorzugsweise 5 bis 45 Mol%, vorzugsweise 5 bis 40 Mol% der Alkylgruppen R in den Trialkylestern der 1,2,4-Cyclohexantripropionsäure nach der Formel (1) 2-Methylbutylreste.

Die erfindungsgemäße Weichmacherzusammensetzung kann zusätzlich noch mindestens einen weiteren Weichmacher enthalten, der aus der Gruppe, bestehend aus Adipaten, Benzoaten, beispielsweise Monobenzoaten oder Glycoldibenzoaten, chlorierten Kohlenwasserstoffen (sog. Chlorparaffinen), Citraten, epoxidierten Fettsäureestern, epoxidierten Pflanzenölen, epoxidierten acylierten Glyceriden, Furandicarboxylaten, Phosphaten, Succinaten, Sulfonamiden, Sulfonaten, Terephthalaten, Isophthalaten, Phthalaten, Trimellitaten, Cyclohexandicarboxylaten und oligomeren oder polymeren Estern auf Basis von Adipin-, Bernstein- oder Sebacinsäure, ausgewählt wird.

In einer bevorzugten Ausführungsform der vorliegenden Erfindung enthält die Weichmacherzusammensetzung mindestens einen weiteren Weichmacher, der aus der Gruppe, bestehend aus Alkylbenzoaten, Alkylsulfonsäureestern des Phenols, Dialkyladipaten, Glycerinestern, C4-bis C6-Alkansäureester von Polyolen, acetylierten oder nicht acetylierten Citronensäuretrialkylestern, Glykoldibenzoaten, Trialkylestern der Trimellitsäure, Dialkylterephthalaten, Dialkylphthalaten, Dialkylisophthalaten, Estern der Furandicarbonsäure, Dialkanoylestern von Dianhydrohexitolen (z.B. Isosorbid), epoxidierten Fettsäurealkylestern, Polymerweichmachern, beispielsweise der Polyadipate, und Dialkylestern der 1,2-, 1,3- oder 1,4-Cyclohexandicarbonsäure, ausgewählt wird.

Die beiden Substanzen, d. h. die Trialkylester der 1,2,4-Cyclohexantripropionsäurekönnen und der weitere Weichmacher, können in unterschiedlichen Mengen in der erfindungsgemäßen Weichmacherzusammensetzung vorhanden sein. Es sollte klar sein, dass die beiden Substanzen in einer Menge vorhanden sein müssen, bei der sie eine Wirkung entfalten, d. h. wo eine weichmachende Wirkung eintritt. In einer bevorzugten Ausführungsform der vorliegenden Erfindung liegen die Verbindung nach Formel (1) und weitere Weichmacher in einem Gewichtsverhältnis (Verbindung nach Formel (1) : weiterer Weichmacher) von 1 : 99 bis 99 : 1, vorzugsweise 10 : 90 bis 90 : 10, besonders bevorzugt 30 : 70 bis 70 : 30 in der Weichmacherzusammensetzung vor.

Die erfindungsgemäße Weichmacherzusammensetzung der vorliegenden Erfindung kann zusätzlich ein epoxidiertes Öl oder einen epoxidierten Alkylester einer Fettsäure enthalten. Epoxidierte Öle oder entsprechende Ester können die thermische Stabilität und die mechanischen Eigenschaften verbessern.

Das epoxidierte Öl kann aus der Gruppe bestehend aus epoxidiertem Sojabohnenöl, epoxidiertem Rizinusöl, epoxidiertem Leinöl, epoxidiertem Palmöl, epoxidiertem Tallöl und Mischungen davon ausgewählt werden. Bevorzugt werden epoxidiertes Sojabohnenöl oder epoxidiertes Leinöl in der erfindungsgemäßen Weichmacherzusammensetzung eingesetzt. Besonders bevorzugt ist epoxidiertes Sojabohnenöl, das auch unter dem Akronym ESBO bekannt ist.

Die epoxidierten Fettsäureester können durch Umesterung der o.g. epoxidierten Öle mit Alkoholen im Bereich von 1 bis 10 Kohlenstoffatomen, bevorzugt 4 bis 9 Kohlenstoffatomen, hergestellt werden. Alternativ kann erst das natürliche Öl umgeestert und die Doppelbindungen der Fettsäure anschließend epoxidiert werden.

Das epoxidierte Öl oder die entsprechenden epoxidierten Fettsäurealkylester können in einer Menge von 1 bis 150 Gewichtsteilen bezogen auf 100 Gewichtsteile der Gesamtsumme aus der Verbindung gemäß Formel (1) und dem weiteren Weichmacher eingesetzt werden. Das epoxidierte Öl oder die epoxidierten Fettsäureester können auch in Mengen von 2 bis 125 Gewichtsteilen, 5 bis 100 Gewichtsteilen, 10 bis 80 Gewichtsteilen oder 20 bis 70 Gewichtsteilen jeweils bezogen auf 100 Gewichtsteile der Gesamtsumme aus der Verbindung gemäß Formel (1) und dem weiteren Weichmacher in der Weichmacherzusammensetzung enthalten sein.

In einer weiterhin bevorzugten Ausführungsform wird der mindestens ein weitere Weichmacher, der in der erfindungsgemäßen Weichmacherzusammensetzung enthalten sein kann, aus der Gruppe, bestehend aus C8- bis C13-Alkylbenzoaten, C4- bis C10-Dialkyladipaten, Pentaerythrit-tetravalerat, C4-bis C10-Trialkyltrimellitaten, C4- bis C9-Dialkylterephthalaten, C4- bis C13-Dialkylphthalaten, insbesondere C9- bis C13-Dialkylphthalaten und C4- bis C10-Dialkylestern der 1,3-Cyclohexandicarbonsäure, ausgewählt.

Ein Gegenstand der vorliegenden Erfindung ist eine Weichmacherzusammensetzung, umfassend Tributylcitrat und Tripentylester der 1,2,4-Cyclohexantripropionsäure nach der oben gezeigten Formel (1), bei denen die drei Alkylgruppen R jeweils 5 Kohlenstoffatome aufweisen, von denen 55 bis 95 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Tripentylcitrat und Tripentylester der 1,2,4-Cyclohexantripropionsäure nach der oben gezeigten Formel (1), bei denen die drei Alkylgruppen R jeweils 5 Kohlenstoffatome aufweisen, von denen 55 bis 95 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Tri-n-hexylcitrat und Tripentylester der 1,2,4-Cyclohexantripropionsäure nach der oben gezeigten Formel (1), bei denen die drei Alkylgruppen R jeweils 5 Kohlenstoffatome aufweisen, von denen 55 bis 95 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Tri-2-ethylhexylcitrat und Tripentylester der 1,2,4-Cyclohexantripropionsäure nach der oben gezeigten Formel (1), bei denen die drei Alkylgruppen R jeweils 5 Kohlenstoffatome aufweisen, von denen 55 bis 95 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Triisononylcitrat und Tripentylester der 1,2,4-Cyclohexantripropionsäure nach der oben gezeigten Formel (1), bei denen die drei Alkylgruppen R jeweils 5 Kohlenstoffatome aufweisen, von denen 55 bis 95 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Acetyltributylcitrat (ATBC) und Tripentylester der 1,2,4-Cyclohexantripropionsäure nach der oben gezeigten Formel (1), bei denen die drei Alkylgruppen R jeweils 5 Kohlenstoffatome aufweisen, von denen 55 bis 95 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Acetyltripentylcitrat und Tripentylester der 1,2,4-Cyclohexantripropionsäure nach der oben gezeigten Formel (1), bei denen die drei Alkylgruppen R jeweils 5 Kohlenstoffatome aufweisen, von denen 55 bis 95 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Diethylhexyladipat (DEHA) und Tripentylester der 1,2,4-Cyclohexantripropionsäure nach der oben gezeigten Formel (1), bei denen die drei Alkylgruppen R jeweils 5 Kohlenstoffatome aufweisen, von denen 55 bis 95 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Diisononyladipat (DINA) und Tripentylester der 1,2,4-Cyclohexantripropionsäure nach der oben gezeigten Formel (1), bei denen die drei Alkylgruppen R jeweils 5 Kohlenstoffatome aufweisen, von denen 55 bis 95 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Diethylhexylterephthalat (DEHT bzw. DOTP) und Tripentylester der 1,2,4-Cyclohexantripropionsäure nach der oben gezeigten Formel (1), bei denen die drei Alkylgruppen R jeweils 5 Kohlenstoffatome aufweisen, von denen 55 bis 95 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Triethylhexyltrimellitat (TEHTM bzw. TOTM) und Tripentylester der 1,2,4-Cyclohexantripropionsäure nach der oben gezeigten Formel (1), bei denen die drei Alkylgruppen R jeweils 5 Kohlenstoffatome aufweisen, von denen 55 bis 95 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Triisononyltrimellitat (TINTM) und Tripentylester der 1,2,4-Cyclohexantripropionsäure nach der oben gezeigten Formel (1), bei denen die drei Alkylgruppen R jeweils 5 Kohlenstoffatome aufweisen, von denen 55 bis 95 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Tripentyltrimellitat (TPTM) und Tripentylester der 1,2,4-Cyclohexantripropionsäure nach der oben gezeigten Formel (1), bei denen die drei Alkylgruppen R jeweils 5 Kohlenstoffatome aufweisen, von denen 55 bis 95 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend den 1,4-Diethylhexylcyclohexandicarbonsäureester (1,4-DEHCH) und Tripentylester der 1,2,4-Cyclohexantripropionsäure nach der oben gezeigten Formel (1), bei denen die drei Alkylgruppen R jeweils 5 Kohlenstoffatome aufweisen, von denen 55 bis 95 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend den 1,2-Diethylhexylcyclohexandicarbonsäureester (1,2-DEHCH) und Tripentylester der 1,2,4-Cyclohexantripropionsäure nach der oben gezeigten Formel (1), bei denen die drei Alkylgruppen R jeweils 5 Kohlenstoffatome aufweisen, von denen 55 bis 95 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend ein Glycoldibenzoat aus der Gruppe, bestehend aus Diethylenglycoldibenzoat, Dipropylenglycoldibenzoat, Triethylenglycoldibenzoat, und Mischungen davon, und Tripentylester der 1,2,4-Cyclohexantripropionsäure nach der oben gezeigten Formel (1), bei denen die drei Alkylgruppen R jeweils 5 Kohlenstoffatome aufweisen, von denen 55 bis 95 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Kunststoffzusammensetzung, die einen Kunststoff und die Weichmacherzusammensetzung, umfassend die Verbindung der Formel (1) und optional einen weiteren Weichmacher enthält. Die Kunststoffzusammensetzung kann zudem das epoxidierte Öl und /oder ein epoxidierten Fettsäurealkylester und/oder mindestens einen zusätzlichen Weichmacher aus der oben genannten Liste enthalten.

Geeignete Kunststoffe sind polymere Substanzen, die vorzugsweise aus der Gruppe, bestehend aus PVC (Polyvinylchlorid), Homo- oder Copolymere auf Basis von Ethylen, Propylen, Butadien, Vinylacetat, Glycidylacrylat, Glycidylmethacrylat, Ethylacrylat, Butylacrylat oder Methacrylat mit Alkoxyresten von verzweigten oder unverzweigten Alkoholen mit einem bis zehn Kohlenstoffatom(en), Acrylnitril oder cyclischen Olefinen, Polyvinylidenchlorid (PVDC), Polyacrylaten, insbesondere Polymethylmethacrylat (PMMA), Polyalkylmethacrylat (PAMA), Polyharnstoffe, silylierte Polymere, Fluorpolymere, insbesondere Polyvinylidenfluorid (PVDF), Polytetrafluorethylen (PTFE), Polyvinylacetat (PVAc), Polyvinylalkohol (PVA), Polyvinylacetale, insbesondere Polyvinylbutyral (PVB), Polystyrolpolymere, insbesondere Polystyrol (PS), expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi und Silikonen, ausgewählt werden.

In einer bevorzugten Ausführungsform ist der Kunststoff in der Weichmacherzusammensetzung ausgewählt aus der Gruppe bestehend aus Polyvinylchlorid (PVC), Polyalkylmethacrylat (PAMA), Polyvinylbutyral (PVB), Polyurethan, Polysulfide, Polymilchsäure (PLA), Polyhydroxybutyral (PHB), Nitrocellulose und Co-Polymeren von Vinylchlorid mit Vinylacetat oder mit Butylacrylat. Besonders bevorzugt ist hiervon PVC als Kunststoff in der erfindungsgemäßen Kunststoffzusammensetzung.

Die Menge an der erfindungsgemäßen Weichmacherzusammensetzung in der Kunststoffzusammensetzung beträgt vorzugsweise 5 bis 150 Gewichtsteile, bevorzugt 10 bis 120 Gewichtsteile, besonders bevorzugt 15 bis 110 Gewichtsteile und ganz besonders bevorzugt 20 bis 100 Gewichtsteile pro 100 Gewichtsteile des Kunststoffs.

Die Kunststoffzusammensetzung kann neben den erwähnten Inhaltsstoffen zusätzliche Additive enthalten. Beispiele für zusätzliche Additive sind rheologische Additive, mit denen die Viskosität der Kunststoffzusammensetzung verringert werden kann. Beispiele für bekannte rheologische Additive sind die unter den Handelsnamen VISCOBYK^{®}-5120, VISCOBYK^{®}-5130 und VISCOBYK^{®}-4041 erhältlichen Produkte. Die Additive können in einem Anteil von 1 bis 12, bevorzugt 2 bis 10 Gewichtsteilen pro 100 Gewichtsteilen PVC in der Kunststoffzusammensetzung enthalten sein.

Darüber hinaus kann die Kunststoffzusammensetzung einen oder mehrere Thermostabilisator(en) enthalten. Geeignete Thermostabilisatoren sind Bleisalze, Organozinnverbindungen, Barium/Zinkverbindungen, Cadmiumverbindungen oder Zinkverbindungen, Calcium/Zink-Stabilisatoren und organisch-basierte Stabilisatoren (sog. OBS). Bevorzugte Thermostabilisatoren sind Barium/Zinkverbindungen, Calcium/Zink-Stabilisatoren und organisch-basierte Stabilisatoren (sog. OBS). Der Anteil des oder der Stabilisatoren in der Kunststoffzusammensetzung beträgt vorzugsweise 1 bis 6 Gewichtsteile pro 100 Gewichtsteilen PVC.

Als weitere Additive können darüber hinaus auch Füllstoffe, Pigmente, Treibmittel und Gleitmittel in der Kunststoffzusammensetzung enthalten sein.

Die erfindungsgemäße Kunststoffzusammensetzung ist vorzugsweise Bestandteil eines Klebstoffs, einer Dichtungsmasse, einer Beschichtungsmasse, eines Lacks, einer Farbe, eines Plastisols, eines Dryblends, eines Schaums, eines Kunstleders, eines Fußbodenbelags, insbesondere dessen Deck- oder Schaumschicht, einer Dachbahn, eines Unterbodenschutzes, einer Gewebebeschichtung, eines Kabels, einer Drahtisolierung, eines Schlauchs, eines Extrusionsartikels, einer Folie, eines Gegenstands im Automobilinnenbereich, einer Tapete, einer Tinte, eines Spielzeugs, einer Kontaktfolie, einer Lebensmittelverpackung oder eines medizinischen Artikels, insbesondere eines Schlauchs oder eines Blutbeutels.

Gegenstand der vorliegenden Erfindung ist deshalb auch die Verwendung der Kunststoffzusammensetzung in Klebstoffen, Dichtungsmassen, Beschichtungsmassen, Lacken, Farben, Plastisolen, Schäumen, Kunstleder, Fußbodenbelägen, insbesondere Deck- und Schaumschicht, Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, insbesondere in Schläuchen oder Blutbeuteln.

Die vorliegende Erfindung wird nachfolgend anhand von Beispielen illustriert. Die folgenden Beispiele sollen die Erfindung erläutern, ohne deren Anwendungsbreite, die sich aus der Beschreibung und den Patentansprüchen ergibt, einzuschränken.

### Beispiele

### Beispiel 1 - Herstellung von Cyclohexan-1,2,4-tripropionsäuretrimethylester (Me-Tc)

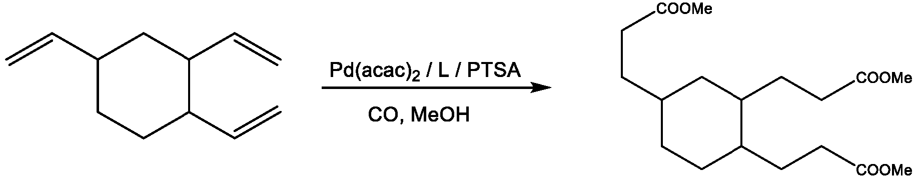

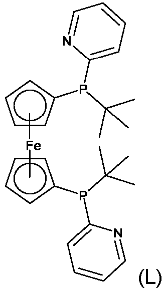

[Pd(acac)2] (15,2 mg, 0,1 mol%), der Katalysator L (103 mg, 0,4 mol%) und p-Toluolsulfonsäure (PTSA) (143 mg, 1,5 mol%) wurden unter einer Argonatmosphäre in einen 100-ml-Stahlautoklaven gegeben. Dann wurden MeOH (30 ml) und Trivinylcyclohexan (8,1 g, 50 mmol) mittels einer Spritze injiziert. Der Autoklav wurde dreimal mit CO gespült und anschließend mit einem CO-Druck von 40 bar beaufschlagt. Die Reaktion erfolgte über 10 h bei 110 °C. Danach wurde der Autoklav auf Raumtemperatur abgekühlt und entspannt. Das gewünschte Produkt wurde durch Destillation (165 °C bei 10⁻³ bar) aufgereinigt und mit ¹H-, ¹³C-NMR und HR-MS charakterisiert (15,6 g, 91% Ausbeute).

### Beispiel 2 - Herstellung verschiedener Cyclohexan-1,2,4-Tripropionsäure-tripentylester (MbPe-Tc)

Der nach Beispiel 1 hergestellte Trimethylester (Me-Tc) wurde in einer typischen Labor-Umesterungsapparatur mit 2-Methylbutanol und n-Pentanol (molares Verhältnis s. Tabelle) zum Produkt MbPe-Tc umgeestert. Die Umesterungsapparatur besteht aus einem Glaskolben, der mit einem Rührer, Temperaturfühler, einer Destillationskolonne, Vakuumteiler und Destillationsaufbau ausgestattet ist.

Zunächst wurden die in Tabelle 1 aufgeführten Mischungen aus n-Pentanol/2-Methylbutanol und M-Tc.Mengen in der Umesterungsapparatur vorgelegt. Im Anschluss wurde die gesamte Apparatur mit Stickstoff gespült. und danach der Tetra(n-butyl)titanat (TNBT) als Katalysator hinzugegeben (0,15 mol% bezogen auf M-Tc). Danach wurde die Reaktion durch Aufheizen gestartet. Die Reaktionstemperatur betrug dabei 130 - 240°C, d. h. sie stieg während der Reaktion an. Methanol, dass bei der Umesterung entstand, wurde über die Kolonne und Destillationsapparatur aus dem System entfernt (65°C Kopftemperatur). Die C5-Alkohole, welche in der Kolonne vom Methanol getrennt wurden, wurden als Rückfluss wieder in das Reaktionsgemisch zurückgeführt. Als kein Destillatanfall mehr zu beobachten war, wurde der Reaktionsfortschritt über eine GC-Analyse kontrolliert Die Reaktion wurde beendet als der Triester >98,8 GC-Flächen% ausmachte, bezogen auf die Summe der Flächen des Edukts, Mono- Di- und Triesters

Im Anschluss an die Reaktion, wurde der überschüssige Alkohol bei 160 °C unter Vakuum abdestilliert. Anschließend wurde die Säurezahl der Reaktionsmischung bestimmt und diese dann durch die Zugabe der 3-fachen stöchiometrischen Menge an 10%-NaOH neutralisiert. Als nächstes wurde die Mischung bei 160 °C mit Stickstoff für 2 h gestrippt. Im letzten Schritt wird das Produkt über einen 0,5 □m PTFE-Filter und Perlite als Filterhilfsmittel klar filtriert.

**Tabelle 1: Pentanolmischungen für die Herstellung der Cyclohexan-1,2,4-Tripropionsäure-tripentylester**

| | Menge M-Tc / g (mol) | Menge n-Pentanol /Gramm (mol) | Menge 2-Methylbutanol / Gramm (mol) | Reinheit Triester (GC, Flächen%) |
|---|---|---|---|---|
| 2a*¹ (Pe/Mb = 50/50) | 1543 (4,5) | 743 (8,4) | 743 (8,4) | 99,0 |
| 2b*² (Pe/Mb = 65/35) | 1710 (5,0) | 1071 (12,2) | 577 (6,6) | 99,3 |
| 2c*² (Pe/Mb = 93/7) | 1883 (5,5) | 1690 (19,2) | 127 (1,4) | 98,9 |

| | | | | |
|---|---|---|---|---|
| *¹ Wie in EP 3 838 888 A1 *² erfindungsgemäß | | | | |

### Beispiel 3 - Herstellung von Plastisolen

Es wurden PVC-Plastisole hergestellt, wie sie beispielsweise zur Fertigung von Deckstrichfilmen für Fußbodenbeläge verwendet werden. Die Angaben in der Plastisolrezeptur sind jeweils in Gewichtsteilen (phr). Die Rezepturen der Polymerzusammensetzung ist in Tabelle 2 aufgelistet.

**Tabelle 2: Übersicht über die hergestellten Plastisole**

| Plastisol | A | B* | C* |
|---|---|---|---|
| | phr | phr | phr |
| PVC (Vestolit^{®} P 1430 K 70 Ultra; Fa. Vestolit) | 100 | 100 | 100 |
| Ester gemäß Beispiel 2a | 50 | | |
| Ester gemäß Beispiel 2b* | | 50 | |
| Ester gemäß Beispiel 2c* | | | 50 |
| epoxidiertes Sojabohnenöl als Co-Stabilisator (Edenol^{®} D81, Fa. Emery) | 3 | 3 | 3 |
| Thermostabilisator auf Ca/Zn-Basis (Reagens MBL 197/9 PF) | 2 | 2 | 2 |

| | | | |
|---|---|---|---|
| * = erfindungsgemäß phr = parts per hundred parts resin | | | |

Zuerst wurden die flüssigen und dann die pulverförmigen Bestandteile in einen PE-Becher eingewogen. Von Hand wurde die Mischung mit einem Spatel so eingerührt, dass kein unbenetztes Pulver mehr vorhanden war. Der verschlossene Becher wurde in die im Speedmixer befindliche Halterung gestellt und vermischt sowie entlüftet. Nach Beenden der Vermischung wurde die Temperatur des Plastisols mit Hilfe eines IR-Thermometers gemessen. Danach wurde das Plastisol sofort für weitere Untersuchungen in einem Klimaschrank bei 25,0 °C temperiert.

### Beispiel 4 - Viskositäten der Plastisole

Die Messung der Viskosität der in Beispiel 3 hergestellten Plastisole wurde mit einem Rheometer Physica MCR 101 (Firma Anton Paar Germany GmbH) mit Hilfe der zugehörigen Software durchgeführt, wobei der Rotationsmodus und das Messsystem CC27 verwendet wurden.

Während der Messung wurden folgende Punkte angesteuert:
- Vorscherung von 100 s-1 für einen Zeitraum von 60 Sekunden, bei der keine Messwerte aufgenommen wurden
- Scherraten-Abwärtsrampe von 200 s-1 bis 0,1 s-1. Es wurden 30 Messpunkte aufgenommen mit einer Messpunktdauer von je 10 Sekunden.

Die Messungen wurden bei Raumtemperatur durchgeführt. Die Ergebnisse der Viskositätsmessungen der in Beispiel 3 hergestellten Plastisole mit den jeweils angegebenen Weichmachern bzw. Weichmachermischungen sind in Tabelle 3 gezeigt.

**Tabelle 3: Viskosität der Plastisole in Pa*s nach 24 Stunden, bei einer Schergeschwindigkeit von 200 s⁻¹**

| Rezeptur | Anteil n-Pentanol/2-Methylbutyl gemäß Tabelle 1 / Mol-% | Viskosität nach 24 h / Pa*s |
|---|---|---|
| A | 50/50 | 9,1 |
| B* | 65/35 | 7,8 |
| C* | 93/7 | 6,6 |

| | | |
|---|---|---|
| * erfindungsgemäß | | |

### Beispiel 5 - Gelierung der Plastisole

Die Untersuchung des Gelierverhaltens der Plastisole aus Beispiel 2 wurde mittels Physica MCR 101 im Oszillationsmodus mit einem Platte-Platte Messsystem (PP25), welches schubspannungsgesteuert betrieben wurde, vorgenommen. Eine zusätzliche Temperierhaube wurde an das Gerät angeschlossen, um eine homogene Wärmeverteilung und eine gleichmäßige Probentemperatur zu erreichen.

Folgende Parameter wurden eingestellt:

| | |
|---|---|
| Modus: | Temperatur-Gradient |
| Start-Temperatur: | 25 °C |
| End-Temperatur: | 180 °C |
| Heiz/Kühlrate: | 5 °C/min |
| Oszillations-Frequenz: | 4 bis 0,1 Hz Rampe logarithmisch |
| Kreisfrequenz Omega: | 10 s⁻¹ |
| Anzahl Messpunkte: | 63 |
| Messpunktdauer: | 0,5 min |
| Automatische Spaltnachführung F: | 0 N |
| Konstante Messpunktdauer | |
| Spaltweite | 0,5 mm |

### Durchführung der Messung

Auf die untere Messsystemplatte wurden mit dem Spatel einige Gramm der zu messenden Paste luftblasenfrei aufgetragen. Dabei wurde darauf geachtet, dass nach dem Zusammenfahren des Messsystems etwas Paste gleichmäßig aus dem Messsystem herausquellen konnte (nicht mehr als 6 mm rundum). Der Überschuss wurde mittels Spatel entfernt. Anschließend wurde die Temperierhaube über der Probe positioniert und die Messung gestartet. Bestimmt wurde die komplexe Viskosität der Paste nach 24 Stunden (Lagerung der Paste bei 25 °C in einem Temperierschrank der Fa. Memmert) in Abhängigkeit von der Temperatur.

Als Maß für die Gelierung wurde ein deutlicher Anstieg der komplexen Viskosität betrachtet. Als Vergleichswert wurde daher die Temperatur bei Erreichen einer Pastenviskosität von 1000 Pa*s verwendet. Die erhaltenen Ergebnisse sind in Tabelle 4 aufgeführt.

**Tabelle 4: Gelierung der Plastisole nach 24 Stunden, Temperatur in °C bei Erreichen einer Pastenviskosität von 1000 Pa*s.**

| Rezeptur | Anteil n-Pentanol/2-Methylbutyl gemäß Tabelle 1 in Mol-% | Geliertemperatur [°C] |
|---|---|---|
| A | 50/50 | 77,7 |
| B* | 65/35 | 77,5 |
| C* | 93/7 | 77,3 |

| | | |
|---|---|---|
| * erfindungsgemäß | | |

Bei Einsatz der erfindungsgemäßen Tripentylester kann die Geliertemperatur abgesenkt werden.

### Beispiel 6 - Herstellung von Folien

Die im Beispiel 3 hergestellten Plastisole wurden jeweils zu 1 mm dicken Folien verarbeitet. Dazu wurde zunächst Hochglanztrennpapier (Fa. Sappi, Italien) auf eine Größe von 30 x 44 cm zugeschnitten und im Spannrahmen der Streicheinrichtung LTSV für den Mathis-Ofen eingelegt. Danach wurde der Spannrahmen auf den Führungsrahmen aufgelegt, der Mathis-Ofen (Typ LTF) auf 200 °C eingestellt und der Rahmen nach Erreichen dieser Temperatur 15 Sekunden vorgewärmt. Danach wurde die Rakel in die Einspannvorrichtung gelegt und der Rakelspalt über Vorversuche so eingestellt, dass die Foliendicke nach Abschluss der Gelierung 1 mm (+/- 0,05 mm) betrug. Auf den vorderen Rand des Papiers wurde ein Klebestreifen angebracht, um überschüssiges Plastisol aufzufangen. Danach wurde das Plastisol vor der Rakel aufgetragen und diese durch Ziehen des Führungsrahmens mit der Rakel über das eingespannte Trennpapier verstrichen (3 m/min Geschwindigkeit). Danach wurde die Rakel herausgenommen und der Klebestreifen mit dem überschüssigen Plastisol abgenommen. Anschließend wurde der Spannrahmen in den Ofen eingefahren. Nach der Gelierung (2 Minuten bei 200 °C) wurde der Rahmen wieder aus dem Ofen herausgefahren und nach Abkühlen die Folie von dem Papier abgezogen.

### Beispiel 7 - Bestimmung der Glasübergangstemperatur mittels DMTA

Die Glasüberganstemperatur wurde mit Hilfe von DMTA-Messungen gemäß DIN 65583 mit einem Rheometer der Firma Anton Paar vom Typ MCR 302 bestimmt. Unter konstanten dynamischmechanischen Bedingungen (1 Hz, Deformation 0,3 %) wurden die viskoelastischen Eigenschaften der Folien (1 mm Dicke) in Abhängigkeit der Temperatur (Temperaturrampe von -100 bis +50 °C) erfasst und der Speichermodul, das Verlustmodul und der Verlustfaktor bestimmt. Das Maximum des Verlustmoduls wird dabei als Glasübergangstemperatur ausgewertet. Die nachfolgende Tabelle 5 zeigt jeweils den Mittelwert aus einer Doppelbestimmung.

**Tabelle 5: Massenverlust der Prüfkörper nach drei Tagen bei 120 °C.**

| Foliennr. | Anteil n-Pentanol/2-Methylbutyl gemäß Tabelle 1 in Mol-% | Glasübergangstemperatur der Folien in °C |
|---|---|---|
| A | 50/50 | -28,7 |
| B* | 65/35 | -29,6 |
| C* | 93/7 | -32,3 |

| | | |
|---|---|---|
| * = erfindungsgemäß | | |

Die Glasübergangtemperaturen der erfindungsgemäßen Proben fallen niedriger aus als bei dem Vergleichsbeispiel.

## Patentansprüche

1. Trialkylester der 1,2,4-Cyclohexantripropionsäure nach der Formel (1) 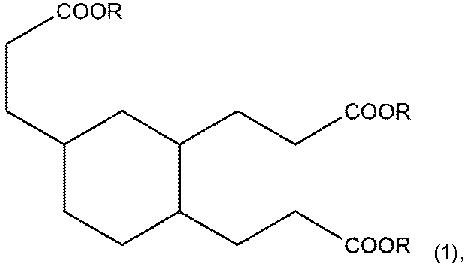 wobei die drei Alkylgruppen R jeweils 5 Kohlenstoffatome aufweisen, von denen 55 bis 95 Mol% n-Pentylreste sind.

2. Trialkylester der 1,2,4-Cyclohexantripropionsäure nach Anspruch 1, wobei 60 bis 95 Mol% der Alkylgruppen R n-Pentylreste sind.

3. Trialkylester der 1,2,4-Cyclohexantripropionsäure nach Anspruch 1 oder 2, wobei 5 bis 45 Mol%, vorzugsweise 5 bis 40 Mol% der Alkylgruppen R 2-Methylbutylreste und/oder 3-Methylbutylreste sind.

4. Trialkylester der 1,2,4-Cyclohexantripropionsäure nach Anspruch 2, wobei 5 bis 45 Mol%, vorzugsweise 5 bis 40 Mol% der Alkylgruppen R 2-Methylbutylreste sind.

5. Weichmacherzusammensetzung, wobei die Weichmacherzusammensetzung die Trialkylester der 1,2,4-Cyclohexantripropionsäure nach einem der Ansprüche 1 bis 4 enthält.

6. Weichmacherzusammensetzung nach Anspruch 5, wobei die Weichmacherzusammensetzung mindestens einen weiteren Weichmacher umfasst, der aus der Gruppe, bestehend aus Alkylbenzoaten, Alkylsulfonsäureestern des Phenols, Dialkyladipaten, Glycerinestern, C4- bis C6-Alkansäureestervon Polyolen, acetylierten oder nicht acetylierten Citronensäuretrialkylestern, Glykoldibenzoaten, Trialkylestern der Trimellitsäure, Dialkylterephthalaten, Dialkylphthalaten, Dialkylisophthalaten, Estern der Furandicarbonsäure, Dialkanoylestern von Dianhydrohexitolen (z.B. Isosorbid), epoxidierten Fettsäurealkylestern, Polymerweichmachern, beispielsweise der Polyadipate, Dialkylestern der 1,2-, 1,3- oder 1,4-Cyclohexandicarbonsäure, ausgewählt wird.

7. Weichmacherzusammensetzung nach einem der vorhergehenden Ansprüche, wobei die Verbindung nach Formel (1) und der weitere Weichmacher (Verbindung nach Formel (1) : weiterer Weichmacher) von 1 : 99 bis 99 : 1, vorzugsweise 10 : 90 bis 90 : 10, besonders bevorzugt 30 : 70 bis 70 : 30 in der Weichmacherzusammensetzung vorhanden sind.

8. Weichmacherzusammensetzung nach einem der vorhergehenden Ansprüche, wobei die Weichmacherzusammensetzung zusätzlich ein epoxidiertes Öl oder einen epoxidierten Alkylester einer Fettsäure enthält.

9. Weichmacherzusammensetzung nach Anspruch 6, wobei das epoxidierte Öl aus der Gruppe bestehend aus epoxidiertem Sojabohnenöl, epoxidiertem Rizinusöl, epoxidiertem Leinöl, epoxidiertem Palmöl, epoxidiertem Stearat, epoxidiertem Oleat, epoxidiertem Tallöl, epoxidiertem Linoleat und Mischungen davon ausgewählt wird.

10. Weichmacherzusammensetzung nach Anspruch 9, wobei das epoxidierte Öl epoxidiertes Sojabohnenöl oder epoxidiertes Leinöl, vorzugsweise epoxidiertes Sojabohnenöl ist.

11. Kunststoffzusammensetzung, umfassend die Weichmacherzusammensetzung nach einem der Ansprüche 1 bis 10 und einen Kunststoff.

12. Kunststoffzusammensetzung nach Anspruch 11, wobei der Anteil der Weichmacherzusammensetzung 5 bis 150 Gewichtsteile pro 100 Gewichtsteile Kunststoff beträgt.

13. Kunststoffzusammensetzung nach Anspruch 11 oder 12, wobei der Kunststoff aus der Gruppe, bestehend aus Polyvinylchlorid (PVC), Polyalkylmethacrylat (PAMA), Polyvinylbutyral (PVB), Polyurethan, Polysulfide, Polymilchsäure (PLA), Polyhydroxybutyral (PHB), Nitrocellulose und Co-Polymeren von Vinylchlorid mit Vinylacetat oder mit Butylacrylat, ausgewählt wird.

14. Kunststoffzusammensetzung nach Anspruch 13, wobei der Kunststoff Polyvinylchlorid (PVC) ist.

15. Verwendung der Kunststoffzusammensetzung nach einem der Ansprüche 11 bis 14 in Klebstoffen, Dichtungsmassen, Beschichtungsmassen, Lacken, Farben, Plastisolen, Schäumen, Kunstleder, Fußbodenbelägen, insbesondere Deck- und Schaumschicht, Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, insbesondere in Schläuchen oder Blutbeuteln.
